# EUROPÄISCHE PATENTSCHRIFT

(11) **EP 2 649 032 B1**
(45) Veröffentlichungstag und Bekanntmachung des Hinweises auf die Patenterteilung: **04.04.2018**
(21) Anmeldenummer: 11788104.5
(22) Anmeldetag: 17.11.2011
(51) Int. Cl.: C07C 45/74, C07C 49/603, C07C 45/82, C07C 45/85

(54) **VERFAHREN ZUR HERSTELLUNG VON ISOPHORON**
METHOD FOR PRODUCING ISOPHORONE
PROCÉDÉ DE PRODUCTION D'ISOPHORONE

(30) Priorität: 08.12.2010 DE 102010062587
(43) Veröffentlichungstag der Anmeldung: 16.10.2013
(73) Patentinhaber: Evonik Degussa GmbH, 45128 Essen (DE)
(72) Erfinder: ORSCHEL, Matthias, 48149 Münster (DE); JANSEN, Robert, 46244 Bottrop (DE); MAIER, Martin, 44625 Herne (DE); GRUND, Gerda, 48653 Coesfeld (DE); SCHWARZ, Markus, 45721 Haltern am See (DE); NITZ, Jörg-Joachim, 46047 Oberhausen (DE); HENGSTERMANN, Axel, 48308 Senden (DE)
(86) Internationale Anmeldenummer: PCT/EP2011/070377
(87) Internationale Veröffentlichungsnummer: WO 2012/076314

(56) Entgegenhaltungen:
- EP-A2- 0 095 783
- US-A- 3 337 423
- US-A- 3 337 632
- US-A- 3 337 633
- DATABASE WPI Week 201014 Thomson Scientific, London, GB; AN 2010-B49244 XP002667094, & CN 101 633 610 A (UNIV ZHEJIANG) 27. Januar 2010 (2010-01-27) in der Anmeldung erwähnt

## Beschreibung

Die Erfindung betrifft ein Verfahren zur Herstellung von Isophoron (3,5,5-Trimethyl-2-cyclohexen-1-on).

Isophoron wird u. a. als hochsiedendes Lösemittel in der Lack-, Druckfarben-, Klebstoff- und der Pflanzenschutzmittel-Industrie verwendet. Nach dem bekannten Stand der Technik kann das Isophoron weiter verarbeitet werden bspw. zu Isophoronnitril, Isophorondiamin, Isophorondiisocyanat oder Keto-Isophoron.

Bei Isophoron handelt es sich um das trimere Kondensationsprodukt des Acetons. Die Herstellung von Isophoron erfolgt über eine katalysierte Aldol-Kondensation von Aceton.

Sowohl die bestehende Patentliteratur als auch die wissenschaftlichen Veröffentlichungen zur Herstellung von Isophoron lassen sich im Wesentlichen in zwei Bereiche einteilen. Man unterscheidet zwischen Flüssigphasen- und Gasphasenprozessen. In CN 101633610A wird auch die Kondensationsreaktion zur Herstellung von Isophoron mit überkritischem Aceton beschrieben.

Während in den beschriebenen Gasphasenprozessen zum größten Teil mit heterogenen, festen Katalysatoren gearbeitet wird, kommen in Flüssigphasenprozessen sowohl homogene als auch heterogene Katalysatorsysteme zum Einsatz.

Die Reaktion in der Flüssigphase wird in der Patentliteratur nahezu ausschließlich unter alkalischen Bedingungen bei erhöhten Temperaturen und hohen Drücken beschrieben.

Auf dem Gebiet der Isophoronchemie sind mehrere Patente der Shell Development Company bekannt (US 2,351,352, US 2,344,226, US 2,399,976, US 2,419,051). Unter anderem wird in der US 2,399,976 ein Kondensationsverfahren zur Herstellung von Isophoron im Kreislaufreaktor mittels Alkalikatalyse beschrieben. Die verwendete Lauge wird in dem Verfahren nach Phasentrennung wieder in den Reaktor zurückgeführt, während das entstandene Reaktionswasser mit der organischen Phase aus dem Reaktorkreislauf entfernt wird.

Des Weiteren ist in US 2,419,051 ein Verfahren beschrieben, in dem durch Hydrolyse der höheren Kondensationsprodukte ein Teil der Überkondensate zurückgespalten werden kann. Die Hydrolyse wird in einem Druckreaktor bei Temperaturen zwischen 130 - 235 °C mit einer erhöhten Laugekonzentration durchgeführt.

Um in der Synthese eine Phasentrennung zu verhindern, und somit eine einphasige Reaktionsführung zu erreichen, sind in den Anmeldungen der Societe Industrielle Des Derivatives De L'Acetylene (DE 10 58 047, GB733650) Alkohole als Lösungsvermittler beschrieben. Dieses Verfahren führt zu einer geringeren Reaktionszeit. Des Weiteren ist dort beschrieben, dass die Rückführung von abgetrennten Nebenprodukten in die Reaktionszone des Reaktors die Selektivität der Isophoronbildung steigert.

In den Patentdokumenten der Hibernia Chemie (DE 10 95 818, DE 11 44 269, DE 12 05 525, DE 11 65 018) aus den 1960er Jahren wird neben dem Einsatz eines einphasigen Edukt / Katalysator-Gemisches mit niedrigen Lauge-Konzentrationen auch die Aufarbeitung mittels Hydrolysekolonne beschrieben. Isophoron wird hier in einem Druckreaktor durch Kondensation von Aceton in flüssiger Phase mittels Alkalimengen (NaOH oder KOH) von weniger als 1 % als Katalysator und unter Anwendung von Wassermengen von unterhalb 20 % bei Temperaturen von 150 - 250 °C hergestellt. Die sich bei der Reaktion ausbildenden zwei Phasen werden sowohl durch eine geeignete Reaktionsführung (Reaktorbau, Impulsgenerator), als auch durch den Einsatz eines Emulgators emulgiert, um einen guten Kontakt zwischen Katalysator und den Reaktanten zu gewährleisten (DE 10 95 818).

Daneben wird in DE 12 05 525 die Aufarbeitung von Nebenprodukten, der sog. Überkondensate beschrieben. Bei 120 - 300 °C findet mit einer wässrigen Alkali-Lösung in einer sogenannten Druckdestillationskolonne unter laufender Entfernung des gebildeten Acetons die Hydrolyse der Überkondensate statt.

Die Gewinnung von reinem Isophoron aus Isophoron-haltigen Kondensationsprodukten gelingt durch eine Abtrennung der Leichtsieder durch Destillation unter demselben Druck, bei dem die Kondensation durchgeführt wird und durch eine weitere Aufarbeitung der noch bestehenden Überkondensate durch Destillation bei vermindertem Druck (DE 11 44 269).

Laut der Anmeldung von BP Chemicals lässt sich durch Verwendung von Kalilauge (KOH) anstelle des sonst üblichen Katalysators Natronlauge (NaOH) die Isophoronausbeute bei gleichbleibender Selektivität um bis zu 7 % steigern (DE 25 20 681).

Weiterhin ist beschrieben, dass die Produktqualität des Isophorons erhöht werden kann, indem man farbbildende Substanzen in einem Seitenstrom aus der Reaktionskolonne ausschleust, und diesen Strom durch Destillation und saure Umsetzung aufreinigt (DE 26 45 281).

Weiterhin existieren Anmeldungen zur Isophoron-Herstellung von Daicel Chemical Industries (JP 8245485, JP 8245486) aus den 1990er Jahren. Diese beschreiben, dass durch Erniedrigung der Wasserkonzentration im Eduktstrom als auch durch Rückführung der wässrigen Laugephase nach der Phasentrennung in den Hydrolyseteil der Reaktivdestillation der Isophoronumsatz gesteigert werden kann.

Neben den bisher genannten Flüssigphasenprozessen mittels homogener Katalysatorsysteme gibt es auch eine Patent-Veröffentlichung mit heterogenen Katalysatorsystemen in der Flüssigphase.

So beschreibt Elf Atochem S. A. in dem Patent US 5,849,957 die Verwendung von Hydrotalciten (Mg₁₋ₓAlₓO₁₊ₓ) als heterogenes Katalysatorsystem für die Herstellung von Isophoron. In diskontinuierlichen Rührkesselversuchen konnten mit einem solchen Katalysator ein Acetonumsatz von 38 % und eine Selektivität zum Isophoron von 51 % erreicht werden.

Im Stand der Technik wird häufig die Herstellung von Isophoron mittels heterogener Katalysatoren auch in der Gasphase beschrieben.

In den Dokumenten der Union Carbide (US 4,086,188, US 4,165,339, EP 095 783) wird die Herstellung von Isophoron mittels Lithium-, bzw. Zink-dotierter Hydrotalcit-artiger Fällungskatalysatoren beschrieben. Mit diesen Katalysatoren kann bei einem Acetonumsatz von 24 % eine Selektivität von 47 % bezüglich Isophoron erreicht werden (US 4,086,188) und der Katalysator durch Abbrennen der Verkokungsrückstände vollständig regeneriert werden (US 4,165,339). Durch Optimierung der Präparationsbedingungen kann die Standzeit eines solchen Katalysators auf bis zu ca. 1000 Stunden erhöht werden (EP 095 783).

In den Patenten der Aristech Chemical Corporation (WO9012645, WO9507255) sind verschiedene oxidische Magnesium/Aluminium-Katalysatoren beschrieben, die durch Aufschlämmung von Pseudoboehmit und Magnesiumoxid hergestellt werden (WO 9012645). Bei einem Aceton Umsatz von 30 % liegt die Selektivität bezüglich Isophoron bei 76 %.

Neben den Katalysatoren beschreibt die Aristech Chemical Company auch ein Verfahren zur Herstellung von Isophoron in der Gasphase in einem Festbettreaktor (WO 95072559). Der Acetonumsatz wird hierbei auf 10 - 35 % begrenzt, um die Bildung von Verkokungsrückständen zu minimieren.

Weiterhin gibt es eine Reihe von Anmeldungen (JP 9059204, JP 9151152, JP 9151153, JP 9157207, JP 9157208, JP 9169687, JP 9169688) von Mitsui Toatsu Chemicals, die verschiedene Zeolith- und Magnesium/Alkalimetall-Katalysatoren für die Herstellung von Isophoron beanspruchen.

In wissenschaftliche Veröffentlichungen ist neben den bereits in den Patenten genannten Katalysatorsystemen ebenfalls die Verwendung von Kohlenstoff-Nanoröhrchen als Katalysator für die Isophoron-Synthese beschrieben. M. G. Stevens (Chem. Commun. 3, 1999) erreicht mit Cäsium-dotierten Kohlenstoff-Nanoröhrchen einen AcetonUmsatz von 11.9 % bei einer Isophoron-Selektivität von 61 %.

Bei der Synthese von Isophoron entsteht eine ganze Reihe von unerwünschten Nebenprodukten. Diese sind bspw. Diacetonalkohol, Mesityloxid, Phoron, Mesitylen sowie eine Reihe von höheren Kondensationsprodukten (Überkondensate) des Acetons (z. B. Xylitone und Isoxylitone). Aus diesem Grund ist die Erzielung von hohen Ausbeuten und Selektivitäten an Isophoron schwierig zu erreichen.

Die technische Aufgabe dieser Erfindung war es daher, ein Verfahren zu finden, das es ermöglicht, die Wirtschaftlichkeit der Isophoron Herstellung zu erhöhen. Dabei sollten auch ökologische Aspekte berücksichtigt sein.

Gegenstand der Erfindung ist ein Verfahren zur Herstellung von Isophoron durch katalysierte Aldol-Kondensationen mit Aceton als Edukt, wobei ein homogener Katalysator eingesetzt wird, Aufarbeitung des Reaktionsproduktes,

Hydrolyse des Wertstroms und Trennung in eine organische und eine wässrige Fraktion, Gewinnung von Isophoron aus der organischen Fraktion, destillative Aufarbeitung der wässrigen Fraktion und Weiterleitung der Brüden vom Kopf der Apparatur der destillativen Aufarbeitung in die Hydrolyse Apparatur wobei das Wasser aus dem Sumpf der destillative Aufarbeitung der wässrigen Fraktion einer Entspannungsverdampfung unterworfen wird und das entstehende gereinigte Wasser in den Prozess zur Herstellung von Isophoron zurückgeführt wird.

Das erfinderische Verfahren kann kontinuierlich, diskontinuierlich oder semikontinuierlich durchgeführt werden. Es wird jedoch in bevorzugter Weise kontinuierlich durchgeführt.

Die Herstellung von Isophoron erfolgt über katalysierte Aldol-Kondensationen mit Aceton als Edukt. Dabei reagieren im ersten Schritt zwei Aceton-Moleküle über das Zwischenprodukt Diacetonalkohol unter Wasserabspaltung zu Mesityloxid. In einer Folgereaktion reagiert das Mesityloxid mit einem weiteren Aceton wiederum unter Wasserabspaltung zum Isophoron. Bei Isophoron handelt es sich also um das Reaktionsprodukt einer Kondensation von drei Molekülen Aceton unter der Abspaltung von zwei Molekülen Wasser.

Als Folge der chemischen Ähnlichkeit des eingesetzten Edukts (Aceton) und der gebildeten (Zwischen-) Produkte verläuft die Isophoron-Synthese nicht allzu selektiv. Aufgrund der Vielzahl von konkurrierenden Aldol-Kondensationsreaktionen erhält man unter Reaktionsbedingungen neben dem gewünschten Zielmolekül Isophoron sowohl eine ganze Reihe von unerwünschten (höheren) Kondensationsprodukten (z. B. Xylitone und Isoxylitone) als auch weitere Nebenkomponenten (z. B. Mesitylen).

Die Isophoron-Synthese ist also durch ein komplexes Reaktionsnetzwerk gekennzeichnet; die Selektivität ist in hohem Maße vom Umsatz abhängig. Um die Bildung von unerwünschten (höheren) Kondensationsprodukten zu minimieren, muss der Acetonumsatz begrenzt werden. Insbesondere in der Gasphasenreaktion kann der verwendete Katalysator durch sich bildende Verkokungsrückstände desaktiviert werden.

Es wurde gefunden, dass das entstehende Reaktionsgemisch durch das erfinderische Verfahren besonders wirtschaftlich und ökologisch zu Isophoron aufgearbeitet werden kann.

Die Kondensationsreaktion von Aceton zu Isophoron (Reaktion) wird bevorzugt in einer katalysierten Flüssigphasenreaktion durchgeführt. Alternativ lässt sich Isophoron auch mittels einer Gasphasenreaktion oder auch durch Reaktion in überkritischem Aceton herstellen.

Für die Durchführung der Reaktion gemäß dem erfindungsgemäßen Verfahren in der Flüssigphase wird das Aceton innerhalb des verwendeten Reaktors durch katalytische Reaktion bei Temperaturen im Bereich von 100 bis 250 °C, bevorzugt 150 - 250 °C, besonders bevorzugt 180 - 250 °C und einem Druckbereich von 5 bis 50 bar, bevorzugt 10 - 50 bar, besonders bevorzugt von 20 - 50 bar zu Isophoron umgesetzt, wobei die angegebenen Werte beliebig miteinander kombiniert werden können.

Für die Durchführung der Reaktion gemäß dem erfindungsgemäßen Verfahren in der Gasphase wird das Aceton innerhalb des verwendeten Reaktors durch katalytische Reaktion bei Temperaturen im Bereich von 100 bis 400 °C, bevorzugt 200 - 400 °C zu Isophoron umgesetzt.

Für die Durchführung der Reaktion gemäß dem erfindungsgemäßen Verfahren im überkritischen Bereich wird das Aceton innerhalb des verwendeten Reaktors durch katalytische Reaktion bei Temperaturen im Bereich von 250 bis 350 °C und einem Druckbereich von 50 bis 200 bar zu Isophoron umgesetzt.

Bei der bevorzugten Reaktion in der Flüssigphase lässt sich Isophoron mittels homogenem Katalysator mit Alkalimengen (NaOH oder KOH) von < 1 Gew.-%, bevorzugt von < 0,5 Gew.-%, besonders bevorzugt < 0,2 Gew.-%, herstellen. Besonders bevorzugt wird als Katalysator NaOH in Mengen von 0,015 bis 0,05 Gew.-% eingesetzt. Die eingesetzte Wasserkonzentration ergibt sich u. a. aus den Rückführströmen der Aufarbeitungsprozesse, sie sollte bezogen auf die gesamte Flüssigkeitsmenge unterhalb < 40 %, bevorzugt < 30 % liegen.

Die Reaktion kann in beliebigen Reaktoren nach dem Stand der Technik, wie z.B. Rohrreaktoren, Rührkesseln, Rührkesselkaskaden, Festbettreaktoren, Druckdestillationsreaktoren bzw. Reaktivdestillationen, mikrostrukturierten Reaktoren, Schlaufenreaktoren usw. oder in Kombinationen aus beliebigen Reaktoren durchgeführt werden. Dabei ist die Wahl der Reaktoren nicht auf die genannte Auswahl beschränkt.

Der Begriff Druckdestillationsreaktor ist hier gleichzusetzen mit Apparaten, in denen eine Reaktivdestillation durchgeführt wird. Die Reaktivdestillation ist in der Fachliteratur hinlänglich beschrieben, z.B. in Ullmann's Encylcopedia of Industrial Chemistry (M. Sakuth, D. Reusch, R. Janowsky: Reactive Distillation © 2008 Wiley-VCH Verlag GmbH & Co KGaA, Weinheim, DOI: 10.1002/14356007.c22_c01.pub2). Hier und in der zitierten Literatur sind alle gängigen Prozesse und Apparate der Reaktivdestillation beschrieben. Wird im folgenden Text der Patentschrift der Begriff der Reaktivdestillationskolonne verwendet, so sind sämtliche Ausführungsformen der Reaktivdestillation, wie in der Literatur beschrieben, gemeint.

In bevorzugter Ausführung erfolgt die Reaktionsdurchführung in Reaktivdestillationskolonnen, Rohrreaktoren oder Festbettreaktoren. Besonders bevorzugt sind Rohrreaktoren.

Nach Durchführung der Reaktion wird das Reaktionsgemisch aufgearbeitet und in die einzelnen Komponenten getrennt. Diese sind neben Isophoron sogenannte Leichtsieder wie z. B. Aceton, Diacetonalkohol und Mesityloxid, sowie eine Reihe von höheren Kondensationsprodukten (Überkondensate) des Acetons (z. B. Xylitone und Isoxylitone), Wasser und gegebenenfalls Katalysator. Dabei wird die Trennung ganz oder teilweise durchgeführt.

Die Abtrennung der einzelnen Fraktionen kann mit allen Trennmethoden wie z. B. Destillation, Entspannungsverdampfung, Kristallisation, Extraktion, Sorption, Permeation, Phasentrennung oder Kombinationen aus den genannten, kontinuierlich oder absatzweise, ein- oder mehrstufig durchgeführt werden. Bevorzugt wird die Trennung durch Destillation in einem oder mehreren Apparaten erreicht.
Dabei kann die Destillation räumlich getrennt von der Isophoronsynthese (Reaktion) durchgeführt werden oder in einem Apparat stattfinden. Bevorzugt erfolgt die Abtrennung der einzelnen Fraktionen durch eine Reaktivdestillation, bevorzugt in einer Reaktivdestillationskolonne.

Besonders bevorzugt wird die Abtrennung räumlich getrennt von der Isophoronsynthese (Reaktion) in einer Reaktivdestillationskolonne mit einer Seitenstromentnahme durchgeführt.

Bevorzugt erfolgt die Abtrennung in drei Fraktionen:
a) Einer Fraktion aus nicht umgesetzten Aceton, Wasser und Leichtsiedern, wie z.B. Diacetonalkohol und Mesityloxid, die kondensiert und anschließend zur Reaktion in den Reaktor zurückgeführt wird.
b) Einer Fraktion, in der insbesondere farbgebende Substanzen angereichert werden. Diese Fraktion wird weiter aufgereinigt und die enthaltenden Wertstoffe werden im Prozess zurückgeführt.
c) Einer Fraktion aus insbesondere Isophoron, höherkondensierten Produkten und Wasser und gegebenenfalls Katalysator, genannt Wertstrom. Diese Fraktion wird anschließend einer Hydrolyse unterworfen.

Die Fraktion a) wird in der bevorzugten Ausführungsform als Brüdenstrom entnommen, enthaltend im Wesentlichen Aceton, Wasser und Leichtsieder, im Wesentlichen Diacetonalkohol und Mesityloxid, kondensiert und dem Reaktor mit den Einsatzstoffen Aceton, Wasser und gegebenenfalls Katalysator wieder zugesetzt.
Die Fraktion b) wird in der bevorzugten Ausführungsform als Seitenstrom der Destillationskolonne, bevorzugt einer Reaktivdestillationskolonne, entnommen, gegebenenfalls neutralisiert und weiter aufgearbeitet. Dabei können bei der Aufarbeitung alle gängigen Trennmethoden wie z.B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich oder absatzweise, ein- oder mehrstufig durchgeführt werden. Bevorzugt wird die Aufreinigung durch Destillation erreicht. Besonders bevorzugt wird die Aufreinigung durch eine Kombination aus Neutralisation oder Extraktion und anschließender Destillation, bevorzugt in einer Reaktivdestillationskolonne, erreicht. Bevorzugt wird die aufgearbeitete Phase mit den Wertprodukten aus Isophoron, Hochsiedern und gegebenenfalls Katalysator in die Hydrolyse geführt. Eine weitere erhaltene Phase aus Wertprodukten, im Wesentlichen enthaltend Aceton, Diacetonalkohol und Mesityloxid, wird bevorzugt in die Reaktion zurückgeführt. Gegebenenfalls anfallende Rückstände werden der thermischen Verwertung zugeführt.

Die Fraktion c) wird einer Hydrolyse unterworfen. Das Ziel der Hydrolyse ist es, Nebenprodukte teilweise oder vollständig in Isophoron, Aceton und andere Wertprodukte zu überführen. Die Hydrolyse kann in allen gängigen Reaktoren, welche bereits oben beschrieben wurden, oder Destillationskolonnen oder Kombinationen aus beiden, durchgeführt werden. Bevorzugt wird die Hydrolyse durch eine Reaktivdestillation durchgeführt, in dem die entstehenden Leichtsieder, im Wesentlichen enthaltend Aceton, Diacetonalkohol und Mesityloxid, direkt aus der Hydrolysezone entfernt und in die Reaktion zurückgeführt werden, und somit nicht mehr für Nebenreaktionen in der Hydrolyse zur Verfügung stehen.

Ganz besonders bevorzugt wird die Hydrolyse der Fraktion c) in einem Apparat, durch eine Reaktivdestillation durchgeführt, bevorzugt in einer Reaktivdestillationskolonne, wobei gleichzeitig die Trennung des Reaktionsgemisches in die Fraktionen a) bis c) erfolgt, so dass die entstehenden Produkte gleichzeitig entsprechend aufgetrennt werden und die Fraktion c) hydrolysiert wird.

Gegebenenfalls können die Hydrolyse und die destillative Abtrennung auch in einem Apparat mit der Isophoronsynthese (Reaktion) stattfinden.

Die Hydrolyse kann in allen Mischungsverhältnissen der organischen Komponenten mit Wasser mit oder ohne Katalysator durchgeführt werden. Die Wasserkonzentration in der Hydrolyse beträgt dabei 0,1 - 99,9 Gew.-%, bevorzugt 30 - 90 Gew.-%. Im Falle der homogenen Katalyse wird bei der Hydrolyse bevorzugt derjenige Katalysator eingesetzt, der auch im Reaktionsteil verwendet wird. Bevorzugt werden Katalysatorkonzentrationen von 0,001 - 10 Gew.-%, besonders bevorzugt von 0,05 - 1 Gew.-%. Der Druck im Hydrolysereaktor beträgt 1-200 bar, bevorzugt 20 - 60 bar, besonders bevorzugt wird die Hydrolyse mindestens bei dem Druck durchgeführt, der auch im Isophoronsyntheseschritt (Reaktion) herrscht. Die Temperatur der Hydrolyse beträgt 100 - 300 °C, bevorzugt 210 - 260 °C. Besonders bevorzugt wird sich bei Verwendung einer Reaktivdestillationskolonne eine Temperatur bzw. ein Temperaturverlauf einstellen, der den Siedetemperaturen im Sumpf und auf den einzelnen Trenn- oder Reaktionsstufen entspricht.

Die Hydrolyse kann in einem oder mehreren Apparaten einstufig oder mehrstufig durchgeführt werden.

Die so aufgearbeitete Fraktion c) wird anschließend aus dem Hydrolysereaktor bzw. der Reaktivdestillationskolonne abgetrennt, abgekühlt und einer Phasentrennung unterworfen.

Die Phasentrennung erfolgt in eine im Wesentlichen organische Fraktion d) und eine im Wesentlichen wässrige Fraktion e), die, bei homogener Katalyse, auch den Katalysator enthält. Dabei können übliche Phasentrennbehälter mit und ohne Einbauten zum Einsatz kommen. Die Phasentrennung erfolgt bei einer Temperatur zwischen 0 - 200 °C, bevorzugt bei 0 - 100 °C, besonders bevorzugt bei 20 - 70 °C und einem Druck von 1 - 150 bar, bevorzugt 20 - 60 bar, besonders bevorzugt bei dem Druck, der auch in der Hydrolyse herrscht.

Die im Wesentlichen organische Fraktion d) mit dem Zielprodukt Isophoron wird gegebenenfalls neutralisiert und mit üblichen Methoden aufgereinigt, so dass ein Isophoron mit der gewünschten Reinheit und Farbstabilität erhalten wird. Dabei können alle gängigen Trennmethoden wie z.B. Destillation, Entspannungsverdampfung, Kristallisation, Extraktion, Sorption, Permeation, Phasentrennung oder Kombinationen aus den genannten zum Einsatz kommen. Die Aufreinigung kann kontinuierlich oder absatzweise, ein- oder mehrstufig, unter Druck oder im Vakuum durchgeführt werden. Bevorzugt wird die Aufreinigung durch Destillation erreicht. Besonders bevorzugt wird die Aufreinigung durch eine Kombination aus Neutralisation oder Extraktion und folgender Destillation erreicht.

An dieser Stelle wird die destillative Aufarbeitung der wässrigen Fraktion e) (Abwasseraufreinigung) und Weiterleitung der Brüden vom Kopf der Apparatur der destillativen Aufbereitung in die Hydrolyseapparatur genauer beschrieben.

Die im Wesentlichen wässrige Fraktion e) wird einer Abwasseraufreinigung zugeführt. Hier erfolgt die Trennung des Reaktionswassers als Hauptbestandteil und gegebenenfalls des Katalysators von gegebenenfalls noch gelösten organischen Komponenten, wie z. B. Isophoron, Aceton und höher kondensierten Produkten. Die Abwasseraufreinigung wird bevorzugt in einer oder mehreren Destillationskolonnen durchgeführt. Erfindungswesentlich ist dabei, dass die Brüden der Abwasserkolonne direkt in den Apparat geleitet werden, in dem die Hydrolyse stattfindet. Dadurch werden mehrere Probleme des derzeitigen Standes der Technik gleichzeitig gelöst:
1) Da die Brüden im Wesentlichen aus Wasser bestehen, stellt sich im Hydrolyseteil eine notwendige, ausreichend hohe Wasserkonzentration ein, so dass kein zusätzliches Frischwasser in die Hydrolyse eingebracht werden muss.
2) Die in der Fraktion e) gelösten, organischen Anteile werden teilweise oder komplett über die Brüden der Abwasserkolonne in den Prozess zurückgeführt. Das minimiert die organische Belastung im Abwasser und, da es sich im Wesentlichen um Isophoron handelt, erhöht die Gesamtausbeute im Prozess. Diese neuartige Verschaltung der Abwasserkolonne trägt somit einen wesentlichen Beitrag zur ökologischen und ökonomischen Prozessführung bei.
3) Außerdem wird die notwendige Wärme für die Hydrolyse bzw. die destillative Trennung des Reaktionsgemisches durch die Brüden zur Verfügung gestellt, es wird keine separate Heizung benötigt.

Der Druck in der Abwasserkolonne beträgt 1 - 200 bar, bevorzugt 20 - 60 bar. Besonders bevorzugt wird bei dem Systemdruck gearbeitet, der sich im Gesamtsystem Hydrolyse/Abwasserkolonne einstellt, wenn die Brüden der Abwasserkolonne direkt in den Hydrolyseteil der Reaktivdestillation geleitet werden. Die Temperatur in der Abwasserkolonne entspricht der Siedetemperatur der Fraktion e) unter den Druckbedingungen. Die bevorzugte Temperatur der Brüden beträgt 200 - 300 °C.

Im Folgenden wird näher beschrieben, wie das Wasser aus dem Sumpf der destillativen Aufarbeitung der wässrigen Fraktion einer Entspannungsverdampfung unterworfen wird und das entstehende gereinigte Wasser in den Prozess zur Herstellung von Isophoron zurückgeführt wird.

Das im Sumpf der Abwasserkolonne anfallende Abwasser (Strom f) kann abgekühlt und verworfen werden. Bevorzugt wird das Abwasser f) aber einer Entspannungsverdampfung zugeführt und somit weiter aufgetrennt. Die Brüden g) der Entspannungverdampfungsstufe, die im Wesentlichen aus reinem Wasser bestehen, können kondensiert und als Wasser in den Prozess, bevorzugt in die Reaktion, z. B. zur Verdünnung des eingesetzten Katalysators (im Falle der homogenen Katalyse) zurückgeführt werden. Dadurch wird die Abwassermenge nochmals reduziert. Die Entspannungsverdampfung kann ein- oder mehrstufig kontinuierlich oder diskontinuierlich durchgeführt werden. Der Druck in der Entspannungsverdampfung liegt in jedem Fall unterhalb des Druckes in der Abwasserkolonne. Bevorzugt ist bei dem erfindungsgemäßen Verfahren die Anwendung einer Entspannungsverdampfung.

Sämtliche Destillations- und Reaktionsschritte im Prozess können in Reaktoren oder Apparaten mit oder ohne Einbauten durchgeführt werden, wie z. B. Dephlegmatoren, ungeordnete Einbauten oder Schüttungen, geordnete Einbauten oder Packungen, Böden mit oder ohne Zwangsführung.

Alle für die Reaktion eingesetzten produktberührten metallischen Werkstoffe und die aus den metallischen Werkstoffen hergestellten Apparate sowie deren Einbauten müssen gegen Laugen beständig sein. Dabei können in Abhängigkeit von der Gefährdung unterschiedliche Beständigkeitsanforderungen bestehen. Für die Beständigkeiten sind nicht nur die chemischen und/oder mechanischen Eigenschaften von Bedeutung, sondern auch die zur Anwendung kommenden Fertigungsmethoden und die Beurteilungsmaßstäbe während der Prüfung.

Für die metallischen Werkstoffe wird zum Teil Bezug auf das AD 2000-Merkblatt HP 0 Ausgabe 11.2008 (Allgemeine Grundsätze für Auslegung, Herstellung und damit verbundene Prüfungen) und DIN EN 10020 Ausgabe 07.2000 (Begriffsbestimmung für die Einteilung der Stähle) genommen. Die dort genannten Werkstoffgruppen werden zitiert, um die Bezeichnungen (z. B. "austenitscher nichtrostender Stahl) zu präzisieren. Sofern technisch sinnvoll, gelten die Aussagen für alle technisch verfügbaren Varianten der Werkstoffe (z. B. Schmiedevarianten, Walzvarianten und Gussvarianten) mit vergleichbarer Beständigkeit gegen Laugekorrosion.
a) Für drucktragende und produktberührte Komponenten können beliebige, nach dem Stand der Technik geeignete Werkstoffe zur Anwendung kommen wie z. B.:
   - Warmfeste Stähle (z. B. Werkstoffuntergruppe 5.1 bis 5.4 und 6.1 bis 6.4 gemäß AD 2000 HP 0)
   - Austenitische nichtrostende Stähle (z. B. Werkstoffuntergruppe 8.1 bis 8.2 gemäß AD 2000 HP 0)
   - Ferritfreie austenitische nichtrostende Stähle (z. B. Werkstoffuntergruppe 8.1 bis 8.2 gemäß AD 2000 HP 0)
   - Ferritisch-austenitische nichtrostende Stähle (z. B. Werkstoffuntergruppe 10.1 bis 10.2 gemäß AD 2000 HP 0)
   - Nickel und Nickellegierungen (z. B. Werkstoffuntergruppe 41 bis 46 gemäß AD 2000 HP 0)

Auch Kombinationen der o. g. Werkstoffe können zur Anwendung kommen. Dabei ist die Wahl der Werkstoffe nicht auf die genannte Auswahl beschränkt und umfasst auch korrosionstechnisch gleichwertige oder höherwertige Varianten. Bevorzugt werden Werkstoffe, die sich nach dem Stand der Technik unter Berücksichtigung der Beanspruchungsbedingungen und Gefährdungen durch eine technische Beständigkeit gegen Laugen auszeichnen. Ein Verzicht auf Wärmebehandlungen ist nicht möglich, wenn sich dadurch die technische Beständigkeit gegen Laugen in unzulässiger Weise ändert.
b) Für nicht drucktragende und produktberührte Komponenten können beliebige, nach dem Stand der Technik geeignete Werkstoffe zur Anwendung kommen wie z. B.:
   - Alle unter a) genannten Werkstoffe
   - Unlegierte Stähle (z. B. Werkstoffuntergruppe 1.1 bis 1.2 gemäß AD 2000 HP 0)
   - Unlegierte Stähle und andere legierte Stähle (z. B. gemäß DIN EN 10020)

   Auch Kombinationen der o. g. Werkstoffe können zur Anwendung kommen. Dabei ist die Wahl der Werkstoffe nicht auf die genannte Auswahl beschränkt und umfasst auch korrosionstechnisch gleichwertige oder höherwertige Varianten. Bevorzugt werden Werkstoffe, die sich nach dem Stand der Technik unter Berücksichtigung der Beanspruchungsbedingungen und Gefährdungen durch eine ausreichende Beständigkeit gegen Laugen auszeichnen. Für nicht drucktragende Komponenten können in Abhängigkeit von der Gefährdung gegebenenfalls temporäre Beständigkeiten in Kauf genommen werden. Ein Verzicht auf Wärmebehandlungen ist nicht möglich, wenn sich dadurch die technische Beständigkeit gegen Laugen in unzulässiger Weise ändert.
c) Die Werkstoffeigenschaften werden durch geeignete Fertigungsverfahren verändert, die im Folgenden nach den in der DIN 8580 Ausgabe 09.2003 (Fertigungsverfahren - Begriffe, Einteilung) genannten Bezeichnungen beschrieben sind. Folgende Fertigungsverfahren können für die Verarbeitung der metallischen Werkstoffe z. B. zur Anwendung kommen:
   - Urformen (z. B. Gießen)
   - Umformen (z. B. Kaltumformen und Warmumformen)
   - Trennen (z. B. Spanen mit geometrisch bestimmter Schneide und Spanen mit geometrisch unbestimmter Schneide)
   - Fügen (z. B. Schmelzschweißen)
   - Beschichten (z. B. Beschichten aus dem flüssigen Zustand, Schmelztauchen, Plattieren, Thermisches Spritzen, Sintern, galvanisches Besichten, chemisches Beschichten und Beschichten aus dem gasförmigen und dampfförmigen Zustand)
   - Stoffeigenschaften ändernde Fertigungsverfahren (Verfestigung durch Umformen wie z. B. Schmieden, Walzen, Strahlen; Wärmebehandeln wie z. B. Vergüten, Rekristallisationsglühen, Spannungsarmglühen, Normalisierungsglühen; Thermomechanische Behandlungen wie z. B. die Kombination von Wärmebehandlung und Umformbehandlung; Sintern und Brennen)

   Auch Kombinationen der o. g. Fertigungsverfahren können zur Anwendung kommen. Dabei ist die Wahl der Fertigungsverfahren nicht auf die genannte Auswahl beschränkt. Bevorzugt werden Verfahren, die nach dem Stand der Technik die erforderliche Laugenbeständigkeit der jeweiligen Werkstoffe und Apparate gewährleisten.
d) Folgende Prüfungen an Apparaten und Einbauten sowie insbesondere an deren Schweißverbindungen können beispielsweise zur Anwendung kommen:
   - Magnetpulverprüfung MT
   - Eindringprüfung PT
   - Durchstrahlungsprüfung RT
   - Ultraschallprüfung UT
   - Sichtprüfung VT
   - Härteprüfung HT
   - Legierungsanalyse

Auch Kombinationen der o. g. Prüfverfahren sind möglich. Dabei ist die Wahl der Prüfverfahren nicht auf die genannte Auswahl beschränkt. Es werden Prüfverfahren und Bewertungsgrundlagen bevorzugt, die nach dem Stand der Technik dazu beitragen, die erforderliche Laugenbeständigkeit der jeweiligen Komponenten zu gewährleisten.

### Beispiel erfindungsgemäß:

Ein Roh-Isophoron-Gemisch bestehend aus Isophoron, leichtersiedenden Komponenten, höhersiedenden Komponenten sowie Wasser und Katalysator, welches auf eine der oben beschriebenen Verfahrensweisen erzeugt wurde, wird dem Hydrolyseapparat entnommen, auf ca. 40 - 60 °C abgekühlt und einer Phasentrennung unterzogen. Das Phasenverhältnis beträgt 1 Teil organische Phase, 4 Teile wässrige Phase. In der wässrigen Phase sind danach noch 1 Gew.- % Isophoron enthalten, dies entspricht etwa 4 Gew.-% der organischen Phase. Bei der nachfolgenden Abwasserdestillation wird der Isophoronanteil nahezu vollständig und das Wasser zu 75 Gew.-% verdampft und in die Hydrolyseapparatur geleitet.
Bei der Abkühlung des Sumpfes der Abwasserkolonne durch Entspannungsverdampfung werden weitere 25 Gew.-% des Wassers zurückgewonnen.

Rechnung für 1 Tonne Isophoronproduktion: 4 Gew.-% (ca. 40 kg) Isophoron Mehrproduktion, Minimierung der Abwassermenge, organische Belastung des Abwassers gering.

### Vergleich, nicht erfindungsgemäß:

Vergleich mit dem herkömmlichen Verfahren: ca. 4 Gew.-% Isophoronverlust über das Abwasser, bezogen auf 1 Tonne Isophoronproduktion. Der Mehrbedarf an Wasser entspricht der 5-fachen Menge, bezogen auf 1 Tonne Isophoronproduktion. Ebenso ist die Abwassermenge 5 mal höher, bezogen auf 1 Tonne Isophoronproduktion.

## Patentansprüche

1. Verfahren zur Herstellung von Isophoron durch katalysierte Aldol-Kondensationen von Aceton als Edukt, wobei ein homogener Katalysator eingesetzt wird,
Aufarbeitung des Reaktionsproduktes,
Hydrolyse des Wertstroms und Trennung in eine organische und eine wässrige Fraktion, Gewinnung von Isophoron aus der organischen Fraktion,
destillative Aufarbeitung der wässrigen Fraktion und Weiterleitung der Brüden vom Kopf der Apparatur der destillativen Aufarbeitung in die Hydrolyseapparatur,
wobei das Wasser aus dem Sumpf der destillativen Aufarbeitung der wässrigen Fraktion einer Entspannungsverdampfung unterworfen wird und das entstehende gereinigte Wasser in den Prozess zur Herstellung von Isophoron zurückgeführt wird.

2. Verfahren zur Herstellung von Isophoron nach Anspruch 1, **dadurch gekennzeichnet, dass** die Durchführung der Reaktion in der Flüssigphase erfolgt, wobei das Aceton durch katalytische Reaktion bei Temperaturen im Bereich von 100 bis 250 °C, bevorzugt 150 - 250 °C, besonders bevorzugt 180 - 250 °C und einem Druckbereich von 5 bis 50 bar, bevorzugt 10 - 50 bar, besonders bevorzugt von 20 - 50 bar zu Isophoron umgesetzt wird, wobei die angegebenen Werte beliebig miteinander kombiniert werden können.

3. Verfahren zur Herstellung von Isophoron nach Anspruch 1, **dadurch gekennzeichnet, dass** die Durchführung der Reaktion in der Gasphase erfolgt, wobei das Aceton durch katalytische Reaktion bei Temperaturen im Bereich von 100 bis 400 °C, bevorzugt 200 - 400 °C zu Isophoron umgesetzt wird.

4. Verfahren zur Herstellung von Isophoron nach Anspruch 1, **dadurch gekennzeichnet, dass** die Durchführung im Überkritischen Bereich erfolgt, wobei das Aceton durch katalytische Reaktion bei Temperaturen im Bereich von 250 bis 350 °C und einem Druckbereich von 50 bis 200 bar zu Isophoron umgesetzt wird.

5. Verfahren zur Herstellung von Isophoron nach mindestens einem der vorherigen Ansprüche, **dadurch gekennzeichnet, dass** die Reaktion in Reaktoren ausgewählt aus Rohrreaktoren, Rührkesseln, Rührkesselkaskaden, Festbettreaktoren, Reaktivdestillationskolonnen, mikrostrukturierten Reaktoren, Schlaufenreaktoren oder in Kombinationen aus diesen Reaktoren, durchgeführt wird.

6. Verfahren zur Herstellung von Isophoron nach mindestens einem der vorherigen Ansprüche, **dadurch gekennzeichnet, dass** nach Durchführung der Reaktion das Reaktionsgemisch aufgearbeitet und in die einzelnen Komponenten getrennt wird, wobei die Abtrennung ganz oder teilweise durchgeführt wird.

7. Verfahren zur Herstellung von Isophoron nach mindestens einem der vorherigen Ansprüche, **dadurch gekennzeichnet, dass** die Abtrennung in drei Fraktionen erfolgt:
a) Einer Fraktion aus nicht umgesetzten Aceton, Wasser und Leichtsiedern, die kondensiert und anschließend zur Reaktion in den Reaktor zurückgeführt wird;
b) Einer Fraktion, in der insbesondere farbgebende Substanzen angereichert werden, wobei diese Fraktion weiter aufgereinigt wird und die enthaltenden Wertstoffe im Prozess zurückgeführt werden;
c) Einer Fraktion aus Isophoron, höherkondensierten Produkten und Wasser und Katalysator, genannt Wertstoffstrom, wobei diese Fraktion anschließend einer Hydrolyse unterworfen wird.

8. Verfahren zur Herstellung von Isophoron nach mindestens einem der vorherigen Ansprüche, **dadurch gekennzeichnet, dass** die Hydrolyse in einer Reaktivdestillationskolonne, durchgeführt wird, in dem die entstehenden Leichtsieder, im Wesentlichen enthaltend Aceton, Diacetonalkohol und Mesityloxid, direkt aus der Hydrolysezone entfernt und in die Reaktion zurückgeführt werden.

9. Verfahren zur Herstellung von Isophoron nach mindestens einem der vorherigen Ansprüche, **dadurch gekennzeichnet, dass** die aufgearbeitete Fraktion c) und einer Phasentrennung unterworfen wird, in eine im Wesentlichen organische Fraktion d) und eine im Wesentlichen wässrige Fraktion e).

10. Verfahren zur Herstellung von Isophoron nach mindestens einem der vorherigen Ansprüche, **dadurch gekennzeichnet, dass** die Aufreinigung der organische Fraktion d) durch Destillation, besonders bevorzugt eine Kombination aus Neutralisation oder Extraktion und folgender Destillation, erfolgt.

11. Verfahren zur Herstellung von Isophoron nach mindestens einem der vorherigen Ansprüche, **dadurch gekennzeichnet, dass** die wässrige Fraktion e) einer Abwasseraufreinigung zugeführt wird, wobei die Abwasseraufreinigung bevorzugt in einer oder mehreren Destillationskolonnen durchgeführt wird.

12. Verfahren zur Herstellung von Isophoron nach mindestens einem der vorherigen Ansprüche, **dadurch gekennzeichnet, dass** der Druck bei der Abwasseraufreinigung in der Abwasserkolonne 1 - 200 bar, bevorzugt 20 - 60 bar, beträgt.

13. Verfahren zur Herstellung von Isophoron nach mindestens einem der vorherigen Ansprüche, **dadurch gekennzeichnet, dass** bei dem Systemdruck in der Abwasserkolonne gearbeitet wird, der sich im Gesamtsystem Hydrolyse/Abwasserkolonne einstellt, bevorzugt wenn die Brüden der Abwasserkolonne direkt in den Hydrolyseteil der Reaktivdestillationskolonne geleitet werden.

## Claims

1. Process for preparing isophorone by catalysed aldol condensations of acetone as a reactant, wherein a homogeneous catalyst is used,
workup of the reaction product,
hydrolysis of the stream of value and separation into an organic fraction and an aqueous fraction, obtaining isophorone from the organic fraction, distillative workup of the aqueous fraction and passing the vapours from the top of the distillative workup apparatus onward into the hydrolysis apparatus, wherein the water from the bottoms of the distillative workup of the aqueous fraction is subjected to a flash evaporation and the purified water which forms is recycled into the process for preparing isophorone.

2. Process for preparing isophorone according to Claim 1, **characterized in that** the reaction is performed in the liquid phase, the acetone being converted to isophorone by catalytic reaction at temperatures in the range from 100 to 250°C, preferably 150 - 250°C and more preferably 180 - 250°C, and a pressure range of 5 to 50 bar, preferably 10 - 50 bar and more preferably of 20 - 50 bar, it being possible to combine the values specified as desired.

3. Process for preparing isophorone according to Claim 1, **characterized in that** the reaction is performed in the gas phase, the acetone being converted to isophorone by catalytic reaction at temperatures in the range from 100 to 400°C and preferably 200-400°C.

4. Process for preparing isophorone according to Claim 1, **characterized in that** it is performed in the supercritical range, the acetone being converted to isophorone by catalytic reaction at temperatures in the range from 250 to 350°C and a pressure range of 50 to 200 bar.

5. Process for preparing isophorone according to at least one of the preceding claims, **characterized in that** the reaction is performed in reactors selected from tubular reactors, stirred tanks, stirred tank cascades, fixed bed reactors, reactive distillation columns, microstructured reactors, loop reactors, or in combinations of these reactors.

6. Process for preparing isophorone according to at least one of the preceding claims, **characterized in that** performance of the reaction is followed by workup of the reaction mixture and separation into the individual components, the removal being performed in full or in part.

7. Process for preparing isophorone according to at least one of the preceding claims, **characterized in that** the removal is effected in three fractions:
a) a fraction composed of unconverted acetone, water and low boilers, which is condensed and then recycled into the reactor for reaction;
b) a fraction in which coloured substances in particular are enriched, this fraction being purified further and the materials of value present being recycled into the process;
c) a fraction composed of isophorone, more highly condensed products and water and catalyst, called material of value stream, this fraction subsequently being subjected to a hydrolysis.

8. Process for preparing isophorone according to at least one of the preceding claims, **characterized in that** the hydrolysis is performed in a reactive distillation column in which the low boilers formed, essentially comprising acetone, diacetone alcohol and mesityl oxide, are removed directly from the hydrolysis zone and recycled into the reaction.

9. Process for preparing isophorone according to at least one of the preceding claims, **characterized in that** the worked-up fraction c) and is subjected to a phase separation into an essentially organic fraction d) and an essentially aqueous fraction e).

10. Process for preparing isophorone according to at least one of the preceding claims, **characterized in that** the organic fraction d) is purified by distillation, more preferably a combination of neutralization or extraction and subsequent distillation.

11. Process for preparing isophorone according to at least one of the preceding claims, **characterized in that** the aqueous fraction e) is supplied to a wastewater cleaning operation, the wastewater cleaning operation preferably being performed in one or more distillation columns.

12. Process for preparing isophorone according to at least one of the preceding claims, **characterized in that** the pressure in the wastewater cleaning operation in the wastewater column is 1 - 200 bar, preferably 20 - 60 bar.

13. Process for preparing isophorone according to at least one of the preceding claims, **characterized in that** operation is effected at the system pressure in the wastewater column which is established in the overall hydrolysis/wastewater column system, preferably when the vapours of the wastewater column are passed directly into the hydrolysis section of the reactive distillation column.

## Revendications

1. Procédé pour la préparation d'isophorone par des condensations aldol catalysées d'acétone comme produit de départ, un catalyseur homogène étant utilisé, traitement du produit de réaction, hydrolyse du flux de substances de valeur et séparation en une fraction organique et une fraction aqueuse, obtention de l'isophorone à partir de la fraction organique, traitement par distillation de la fraction aqueuse et guidage des vapeurs de la tête de l'appareil de traitement par distillation dans l'appareil d'hydrolyse, l'eau provenant du fond du traitement par distillation de la fraction aqueuse étant soumise à une évaporation par détente et l'eau purifiée formée étant recyclée dans le procédé pour la préparation d'isophorone.

2. Procédé pour la préparation d'isophorone selon la revendication 1, **caractérisé en ce que** la réalisation de la réaction a lieu en phase liquide, l'acétone étant transformée en isophorone par réaction catalytique à des températures dans la plage de 100 à 250°C, de préférence de 150-250°C, de manière particulièrement préférée de 180-250°C et dans une plage de pression de 5 à 50 bars, de préférence de 10-50 bars, de manière particulièrement préférée de 20-50 bars, les valeurs indiquées pouvant être combinées de manière quelconque les unes avec les autres.

3. Procédé pour la préparation d'isophorone selon la revendication 1, **caractérisé en ce que** la réalisation de la réaction a lieu en phase gazeuse, l'acétone étant transformée en isophorone par réaction catalytique à des températures dans la plage de 100 à 400°C, de préférence de 200-400°C.

4. Procédé pour la préparation d'isophorone selon la revendication 1, **caractérisé en ce que** la réalisation a lieu dans la plage surcritique, l'acétone étant transformée en isophorone par réaction catalytique à des températures dans la plage de 250 à 350°C et dans une plage de pression de 50 à 200 bars.

5. Procédé pour la préparation d'isophorone selon au moins l'une quelconque des revendications précédentes, **caractérisé en ce que** la réaction est réalisée dans des réacteurs choisis parmi les réacteurs tubulaires, les cuves agitées, les cascades de cuves agitées, les réacteurs à lit fixe, les colonnes de distillation réactive, les réacteurs microstructurés, les réacteurs à boucle à circulation ou dans des combinaisons de ces réacteurs.

6. Procédé pour la préparation d'isophorone selon au moins l'une quelconque des revendications précédentes, **caractérisé en ce qu'**après la réalisation de la réaction, le mélange réactionnel est traité et séparé en composants individuels, la séparation étant réalisée totalement ou partiellement.

7. Procédé pour la préparation d'isophorone selon au moins l'une quelconque des revendications précédentes, **caractérisé en ce que** la séparation a lieu en trois fractions :
a) une fraction constituée par de l'acétone non transformée, de l'eau et les substances légères, qui est condensée et ensuite recyclée pour la réaction dans le réacteur ;
b) une fraction dans laquelle des substances colorantes sont en particulier enrichies, cette fraction étant purifiée davantage et les substances de valeur contenues étant recyclées dans le procédé ;
c) une fraction constituée par l'isophorone, des produits à condensation supérieure, de l'eau et du catalyseur, appelée flux de substances de valeur, cette fraction étant ensuite soumise à une hydrolyse.

8. Procédé pour la préparation d'isophorone selon au moins l'une quelconque des revendications précédentes, **caractérisé en ce que** l'hydrolyse est réalisée dans une colonne de distillation réactive, dans laquelle les substances légères formées, contenant essentiellement de l'acétone, de l'alcool diacétonique et de l'oxyde de mésityle, sont éliminées directement de la zone d'hydrolyse et recyclées dans la réaction.

9. Procédé pour la préparation d'isophorone selon au moins l'une quelconque des revendications précédentes, **caractérisé en ce que** la fraction c) traitée et est soumise à une séparation des phases en une fraction d) essentiellement organique et une fraction e) essentiellement aqueuse.

10. Procédé pour la préparation d'isophorone selon au moins l'une quelconque des revendications précédentes, **caractérisé en ce que** la purification de la fraction d) organique a lieu par distillation, de manière particulièrement préférée par une combinaison d'une neutralisation ou d'une extraction et d'une distillation consécutive.

11. Procédé pour la préparation d'isophorone selon au moins l'une quelconque des revendications précédentes, **caractérisé en ce que** la fraction e) aqueuse est introduite dans une purification des eaux usées, la purification des eaux usées étant de préférence réalisée dans une ou plusieurs colonnes de distillation.

12. Procédé pour la préparation d'isophorone selon au moins l'une quelconque des revendications précédentes, **caractérisé en ce que** la pression lors de la purification des eaux usées dans la colonne des eaux usées est de 1-200 bars, de préférence de 20-60 bars.

13. Procédé pour la préparation d'isophorone selon au moins l'une quelconque des revendications précédentes, **caractérisé en ce qu'**on travaille dans la colonne des eaux usées à la pression de système qui se règle dans le système global hydrolyse/colonne des eaux usées, de préférence lorsque les vapeurs de la colonne des eaux usées sont guidées directement dans la partie hydrolyse de la colonne de distillation réactive.
